(19) Europäisches Patentamt
European Patent Office
Office européen des brevets

(11) Publication number: 0 092 591
B1

(12) **EUROPEAN PATENT SPECIFICATION**

(45) Date of publication of patent specification: **01.10.86**

(51) Int. Cl.⁴: **C 07 C 76/06**, C 07 C 79/12 // C07C87/62

(21) Application number: **82103407.1**

(22) Date of filing: **22.04.82**

# (54) Process for preparing 1-chloro-2,6-dinitro-4-(trifluoromethyl) benzene free from nitrosating agents.

(43) Date of publication of application:
**02.11.83 Bulletin 83/44**

(45) Publication of the grant of the patent:
**01.10.86 Bulletin 86/40**

(84) Designated Contracting States:
**AT BE CH DE FR GB LI LU NL SE**

(56) References cited:
**EP-A-0 019 281**
**EP-A-0 022 241**
**US-A-2 040 123**
**US-A-3 956 409**
**US-A-4 120 905**

(73) Proprietor: **OXON ITALIA S.p.A.**
**Via Manzoni, 44**
**I-20121 Milano (IT)**

(72) Inventor: **Pallucca, Edoardo, Dr.**
**Via della Libertà 47**
**I-20019 Settimo Milanese Milano (IT)**

(74) Representative: **Vatti, Paolo, Dr. Ing. et al**
**Fumero - Studio Consulenza Brevetti**
**Widenmayerstrasse 4/I**
**D-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 092 591 B1**

Courier Press, Leamington Spa, England.

**Description**

This invention relates to a process for preparing 1-chloro-2,6-dinitro-4-(trifluoromethyl) benzene free from nitrosating agnets, from which it is possible to prepare technical TRIFLURALIN with a particularly low nitrosoamine content (specifically NDPA).

It is well known that certain agricultural products can contain nitrosoamines, and the danger of these substances are equally well known.

In particular, it has been public knowledge since 1976 that nitrosoamines are present in formulations of agricultural products such as TRIFLURALIN

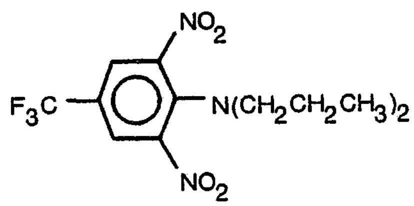

which is a very important selective herbicide, and is prepared by the following reaction

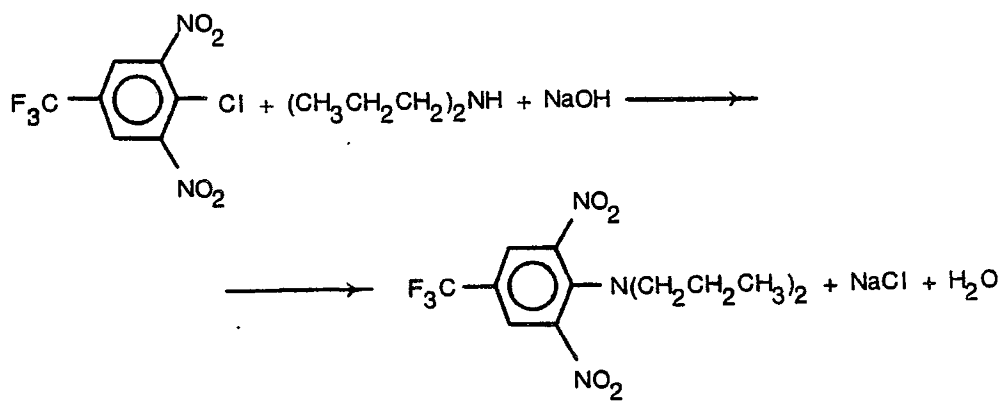

This compound contains N-nitrosodipropylamine (NDPA), i.e. $(CH_3CH_2CH_2)_2N—NO$ as impurity, this forming during the herbicide synthesis.

This is because 1-chloro-2,6-dinitro-4-(trifluoromethyl) benzene (DINITRO-PCBT), which is prepared by dinitrating 1-chloro-4-(trifluoro-methyl) benzene (PCBT), and which is the starting substance for synthesising TRIFLURALIN, contains dissolved nitrogen oxides of various composition, which are responsible for the formation of nitrosoamines in accordance with the reaction $(CH_3CH_2CH_2)_2NH + (NO_x \rightarrow (CH_3CH_2CH_2)N—NO$ which takes place in a basic environment.

As stated the danger of nitrosoamines is well known. They exercise an acute hepatotoxic action, and in particular are carcinogenic, mutagenic and teratogenic. It is therefore essential that the quantity of NDPA in the TRIFLURALIN is as low as possible, and legislation in the more advanced countries considers that an NDPA content in the technical product of less than 1 ppm is acceptacle.

From research carried out up to the present time, it has emerged that if the DINITRO-PCBT is synthesised without special purification processes, the quantity of dissolved nitrogen oxides is such as to generate an DPNA content of between 150 and 500 pm in the technical TRIFLURALIN subsequently produced. If purification processes are introduced (for example those indicated in USA patent No. 4,120,905), the nitrosating agents contained in the DINITRO-PCBT are reduced, but the results obtained are not constant and it is not possible to obtain tehcnical TRIFLURALIN with an NDPA content of less than 1 ppm.

Research has therefore been directed towards methods which provide for eliminating the formed nitrosoamines from the final product.

Even if they allow a final product of satisfactory characteristics to be obtained, processes which comprise the destruction of preformed nitrosoamines have the very serious drawback of being dangerous because of the presence of nitrosoamines in certain stages of the process during which contact, including accidental contact, is always possible with the environment and with the process operators.

It has therefore appeared very desirable to discover processes which exclude the formation of nitrosoamines in order to use them in place of processes which destroy the already formed nitrosoamines, and the research underlying the invention has developed in this direction.

In EP—A—0 022 241 and EP—A—0 019 281 there are described plain methods to eliminate the nitrosating agents from DINITRO-PCBT. To do so, a mechanical filtering takes place which is in so far rather

complicated since a solid product must be filtered and consequently the same product from the filter must be recovered. This method has certain drawbacks particularly because the periodical cleaning of the filtering cloths makes it more or less unavoidable that the operators come in to contact with highly toxic products involved. Furthermore the nitrosoamine content of the final product is not so low as one would like it to have.

The aforementioned research, however, has been successful, and the present invention therefore relates to the original purification process for obtaining DINITRO-PCBT practically free from nitrosating agents, to which said research has led. As will be apparent hereinafter, using the DINITRO-PCBT obtained by this process it is possible to prepare technical TRIFLURALIN with an NDPA content which is always less than 1 ppm. Thus the stated object is completely attained.

The present invention therefore relates to a process for preparing 1-chloro-2,6-dinitro-4-(trifluoromethyl)benzene (DINITRO-PCBT) free from nitrosating agents, characterised in that DINITRO-PCBT is treated with an aqueous bisulphite solution having an $SO_2$ concentration of between 1% and 5% at a pH of between 1 and 3, at a temperature of between 50° and 100°C for a time of between 1 and 3 hours, and in that the organic layer is separated from the aqueous layer, and the acidity and last traces of the bisulphite solution are eliminated from the organic layer by an aqueous alkaline solution.

The bisulphite used is preferably sodium bisulphite, but analogous results can be obtained by using other bisulphites such as potassium bisulphite or ammonium bisulphite. Alternatively, the bisulphite can be prepared directly in the reactor by reacting $SO_2$ with the necessary quantity of alkali. As stated, the $SO_2$ concentration of the bisulphite solution is between 1% and 5%. Preferably, a concentration of 1.6% is used. Concentrations higher than 5% should be avoided, because they decrease the yield without increasing purity.

The pH is preferably kept at around 2, and must not exceed 3 in order not to reduce the yield (as occurs for example if sodium sulphite is used instead of the bisulphite) because high pH values favour substitution of the mobile Cl of the 1-chloro-2,6-dinitro-4-(trifluoromethyl)benzene. Whereas pH values which are too low favour $SO_2$ elimination, and thus make the treatment ineffective. In this respect, the initial DINITRO-PCBT must not contain mineral acids (such as $H_2SO_4$ or $HNO_3$), which can be eliminated by neutralisation.

The temperature is preferably kept at 70°—75°C. In this respect it is important not to fall below the melting point of the DINITRO-PCBT whereas too high temperatures favour yield reduction.

The reaction time is preferably 2 hours. Very long reaction times favour yield reduction.

The choice of the alkali for eliminating the acidity and last traces of the bisulphite-contaminated solution contained in the treated organic DINITRO-PCBT layer is not very important. $Na_2SO_3$ is preferably used for economical reasons and because the pH of the aqueous solution is not too high, so that there is no appreciable yield reduction.

Some examples of how the process according to the invention can be carried out in practice are given hereinafter. These are obviously purely indicative, and in no way limit the invention.

Example 1

200 ml of $H_2O$ and 100 g of 1-chloro-2,6-dinitro-4-(trifluoromethyl)benzene (DINITRO-PCBT) are fed into a reactor. The mass is heated to 70°—75°C under strong stirring in order to obtain perfect mixing of the organic layer with the aqueous layer, after which 13.3 g of a sodium bisulphite solution containing 25.5% of $SO_2$ (corresponding to an overall concentration of 1.59% if the $H_2O$ present in the mass is taken into account) are dripped in over about 20 minutes.

The mass is kept at 70°—75° for 2 hours under continuous vigorous stirring. The stirring is stopped, and the mass allowed to decant. The lower organic layer is separated, and is added to a solution containing 200 ml of $H_2O$ and 5 g of $Na_2CO_3$ which has previously been heated to 70°—75°C. The mass is stirred vigorously for 30—60 minutes, after which stirring is stopped.

The lower organic layer is separated, to give 96 g of purified DINITRO-PCBT.

The following results are obtained in converting samples of the initial DINITRO-PCBT and of the purified DINITRO-PCBT into TRIFLURALIN and analysing the products obtained foir nitrosoamine: using the initial DINITRO-PCBT 156 ppm NDPA present in the TRIFLURALIN produced using purified DINITRO-PCBT 0.5 ppm NDPA present in the TRIFLURALIN produced.

As can be seen, the NDPA content of the TRIFLURALIN produced using the DINITRO-PCBT prepared by the process according to the present invention is extremely low, and equal to one half the maximum allowable value under the most severe present legislation.

Example 2

The procedure of example 1 is followed, but using 26.6 g of a sodium bisulphite solution containing 25.5% of $SO_2$.

In this manner, 93 g of purified DINITRO-PCBT are obtained.

The NDPA content of the TRIFLURALIN produced is 0.45 ppm in this case.

Example 3

The procedure of example 1 is followed, but using 6.4 g of potassium metabisulphite containing 53% of $SO_2$ dissolved in 10 ml of $H_2O$.

95 g of purified DINITRO-PCBT are obtained.

The NDPA content of the TRIFLURALIN produced is 0.7 ppm.

Example 4

The procedure of example 1 is followed, but 3.3 g of sodium sulphite dissolved in 10 ml of $H_2O$ are used.

87 g of purified DINITRO-PCBT are obtained.

In this case, the two high pH value leads to an unacceptable yield reduction.

Example 5

The procedure of example 1 is followed, but using 6.1 g of a sodium bisulphite solution containing 25.5% of $SO_2$.

97 g of purified DINITRO-PCBT are obtained.

The NDPA content of the TRIFLURALIN produced is 0.9 ppm.

Example 6

The procedure of example 1 is followed, but using 8 g of 30 % NaOH for the alkalising stage.

88 g of DINITRO-PCBT are obtained.

Again, the too high pH value lead to an unacceptable yield reduction.

**Claims**

1. A process for preparing 1-chloro-2,6-dinitro-4-(trifluoromethyl)benzene (DINITRO-PCBT) free from nitrosating agents, characterised in that DINITRO-PCBT is treated with an aqueous bisulphite solution having an $SO_2$ concentration of between 1% and 5% at a pH of between 1 and 3, at a temperature of between 50° and 100° for a time of between 1 and 3 hours, and in that the organic layer is separated from the aqueous layer, and the acidity and last traces of the bisulphite solution are eliminated from the organic layer by an aqueous alkaline solution.

2. A process as in claim 1, wherein sodium bisulphite is used for the DINITRO-PCBT treatment solution.

3. A process as in claim 1, wherein potassium or ammonium bisulphite are used for the DINITRO-PCBT treatment solution.

4. A process as in claim 1, wherein the bisulphite for treating the DINITRO-PCBT is prepared directly in the reactor by reacting $SO_2$ with alkali.

5. A process as in claims 1 to 4, wherein the aqueous bisulphite solution has an $SO_2$ concentation of 1.6%.

6. A process as in claims 1 to 5, wherein the pH is kept at around 2.

7. A process as in claims 1 to 6, wherein the temperature is kept between 70° and 75°C.

8. A process as in claims 1 to 7, wherein the reaction time is kept equal to about 2 hours.

9. A process as in claims 1 to 8, wherein sodium carbonate is used to form the alkaline solution for neutralising the organic layer.

**Patentansprüche**

1. Verfahren zum Darstellen von 1-Chlor-2,6-dinitro-4-(trifluoromethyl)benzol (DINITRO-PCBT), frei von Nitrosierungsmitteln, dadurch gekennzeichnet, daß DINITRO-PCBT bei einer Temperatur von 50° und 100°C über einen Zeitraum von zwischen 1 und 3 Stunden mit einer wäßrigen Hydrogensulfitlösung behandelt wird, die eine $SO_2$-Konzentration von zwischen 1% und 5% bei einem pH von zwischen 1 und 3 aufweist, und daß die organische Schicht von der wäßrigen Schicht abgetrennt wird und die Acidität und letzte Spuren de Hydrogensulfitlösung mit einer wäßrigen alkalischen Lösung aus der organischen Schicht entfernt werden.

2. Verfahren wie in Anspruch 1, wobei Natriumhydrogensulfit für die DINITRO-PCBT-Behandlungslösung verwendet wird.

3. Verfahren wie in Anspruch 1, wobei Kalium - oder Ammoniumhydrogensulfit für die DINITRO-PCBT-Behandlungslösung verwendet werden.

4. Verfahren wie in Anspruch 1, wobei das Hydrogensulfat zum Behandeln des DINITRO-PCBT durch Umsetzen von $SO_2$ mit Alkali direkt im Reaktor dargestellt wird.

5. Verfahren wie in den Ansprüchen 1—4, wobei die wäßrige Hydrogensulfitlösung eine $SO_2$-Konzentration von 1,6% aufweist.

6. Verfahren wie in den Ansprüchen 1—5, wobei der pH bei etwa 2 gehalten wird.

7. Verfahren wie in den Ansprüchen 1—6, wobei die Temperatur zwischen 70° und 75° C gehalten wird.

8. Verfahren wie in den Ansprüchen 1—7, wobei die Reaktionszeit bei etwa 2 Stunden gehalten wird.

9. Verfahren wie in den Ansprüchen 1—8, wobei Natrium-carbonat verwendet wird, um die alkalische Lösung zum Neutralisieren der organischen Schicht herzustellen.

**Revendications**

1. Procédé de préparation de 1-chloro-2,6-dinitro-4-(trifluorométhyl)benzène (DINITRO-PCBT) exempt d'agents de nitrosation, caractérisé en ce que le DINITRO-PCBT est traité avec une solution aqueuse de bisulfute ayant une concentration en $SO_2$ entre 1% et 5%, à un pH entre 1 et 3, à une température entre 50° et 100° pendant une durée de 1 à 3 heures et en ce que la couche organique est séparée de la couche aqueuse et que l'acidité et les dernières traces de la solution de bisulfite sont éliminées de la couche organique par une solution alcaline aqueuse.

2. Procédé selon la revendication 1, dans lequel on utilises du bisulfite de sodium pour la solution de traitement du DINITRO-PCBT.

3. Procédé selon la revendication 1, dans lequel on utilise du bisulfite de potassium ou d'ammonium pour la solution de traitement du DINITRO-PCBT.

4. Procédé selon la revendication 1, dans lequel le bisulfite pour traiter le DINITRO-PCBT est préparé directement dans le réacteur par réaction de $SO_2$ avec un alcali.

5. Procédé selon les revendications 1 à 4, dans lequel la solution aqueuse de bisulfite a une concentration en $SO_2$ de 1,6%.

6. Procédé selon les revendications 1 à 5, dans lequel le pH est maintenu autour de 2.

7. Procédé selon les revendications 1 à 6, dans lequel la température est maintenue entre 70° et 75°C.

8. Procédé selon les revendications 1 à 7, dans lequel la durée de réaction est maintenue égale à environ 2 heures.

9. Procédé selon les revendications 1 à 8, dans lequel on utilise du carbonate de sodium pour former la solution alcaline pour la neutralisation de la couche organique.